# EUROPEAN PATENT APPLICATION

(11) **EP 4 389 890 A1**
(43) Date of publication of application: **26.06.2024**
(21) Application number: 22873042.0
(22) Date of filing: 26.09.2022
(51) Int. Cl.: C12N 15/11, C07K 1/02, C07K 1/14, C12P 21/02, C12Q 1/6869, C40B 40/08, C40B 40/10

(54) **TRNA, AMINOACYL-TRNA, POLYPEPTIDE SYNTHESIS REAGENT, UNNATURAL AMINO ACID INCORPORATION METHOD, POLYPEPTIDE PRODUCTION METHOD, NUCLEIC ACID DISPLAY LIBRARY PRODUCTION METHOD, NUCLEIC ACID/POLYPEPTIDE CONJUGATE, AND SCREENING METHOD**

(30) Priority: 24.09.2021 JP 2021156183
(71) Applicant: FUJIFILM Corporation, Tokyo 106-8620 (JP)
(72) Inventor: ISHII, Yukiko, Ashigarakami-gun, Kanagawa 258-8577 (JP); TOMINAGA, Jo, Ashigarakami-gun, Kanagawa 258-8577 (JP); KIHARA, Shiori, Ashigarakami-gun, Kanagawa 258-8577 (JP); HOHSAKA, Takahiro, Nomi-shi, Ishikawa 923-1292 (JP); WATANABE, Takayoshi, Nomi-shi, Ishikawa 923-1292 (JP)
(74) Representative: Hoffmann Eitle
(86) International application number: PCT/JP2022/035791
(87) International publication number: WO 2023/048291

(57) **Abstract**

Provided are a tRNA obtained by modifying a tRNA for tryptophan of Mycoplasma pneumoniae, the tRNA including a UUA as an anticodon to pair with a UAA codon, and an application of the tRNA.

## Description

### BACKGROUND OF THE INVENTION

### 1. Field of the Invention

The present disclosure relates to a tRNA, an aminoacyl tRNA, a reagent for polypeptide synthesis, an introduction method of an unnatural amino acid, a production method of a polypeptide, a production method of a nucleic acid display library, a nucleic acid-polypeptide conjugate, and a screening method.

### 2. Description of the Related Art

A polypeptide into which an unnatural amino acid (also referred to as a special amino acid) is introduced has attracted attention as a candidate for a pharmaceutical product.

JP2012-058092A discloses a method in which a tRNA is acylated with a special amino acid by using an artificial aminoacylation catalyst, a library of a special polypeptide is produced by a cell-free translation system including the tRNA acylated with the special amino acid, and the special polypeptide bound to a target protein is screened.

WO2018/174078A discloses a production method and a screening method of a cyclic peptide compound having excellent cell membrane permeability.

WO2007/055429A discloses a tRNA having a CUA as an anticodon to pair with a UAG codon, which is obtained by modifying tRNA for tryptophan of Mycoplasma capricolum, and a method of introducing an unnatural amino acid into a polypeptide by using the tRNA.

### SUMMARY OF THE INVENTION

An object of the present disclosure is to provide a tRNA which translates a UAA codon into an amino acid, and an aminoacyl tRNA; a reagent for polypeptide synthesis, which introduces at least two types of unnatural amino acids into a polypeptide, an introduction method of an unnatural amino acid, a production method of a polypeptide, and a production method of a nucleic acid display library; a nucleic acid-polypeptide conjugate in which at least two types of unnatural amino acids are introduced into a polypeptide; and a screening method of finding a polypeptide having a target activity from polypeptides into which at least two types of unnatural amino acids are introduced.

The specific methods for achieving the object include the following aspects.
<1> A tRNA obtained by modifying a tRNA for tryptophan of Mycoplasma pneumoniae, the tRNA comprising a UUA as an anticodon to pair with a UAA codon.
<2> The tRNA according to <1>, in which a base adjacent to a 5' side of a CCA terminal is A or G.
<3> The tRNA according to <1> or <2>, in which a combination of a base at a third position from a 5' terminal and a base paired with the base is UA, GU, or UG.
<4> The tRNA according to <1> or <2>, in which a combination of a base at a fourth position from a 5' terminal and a base paired with the base is GU.
<5> A tRNA comprising a base sequence set forth in one selected from the group consisting of SEQ ID NO: 7, SEQ ID NO: 8, SEQ ID NO: 9, SEQ ID NO: 13, SEQ ID NO: 15, and SEQ ID NO: 17.
<6> An aminoacyl tRNA in which an amino acid is bonded to the tRNA according to any one of <1> to <5>.
<7> The aminoacyl tRNA according to <6>, in which the amino acid is one selected from the group consisting of an unnatural amino acid, a modified amino acid, and derivatives of the unnatural amino acid and the modified amino acid.
<8> The aminoacyl tRNA according to <6>, in which the amino acid is one selected from the group consisting of chloroacetyl lysine, N-methylalanine, N-methylphenylalanine, and a fluorescent-labeled amino acid.
<9> A reagent for polypeptide synthesis comprising
   a tRNA having CUA as an anticodon to pair with a UAG codon, the tRNA being obtained by modifying a tRNA for tryptophan of Mycoplasma capricolum, and
   a tRNA having UUA as an anticodon to pair with a UAA, the tRNA being obtained by modifying a tRNA for tryptophan of Mycoplasma pneumoniae.
<10> A reagent for polypeptide synthesis comprising
   an aminoacyl tRNA in which a first unnatural amino acid is bonded to a tRNA having CUA as an anticodon to pair with a UAG codon and being obtained by modifying a tRNA for tryptophan of Mycoplasma capricolum, and
   an aminoacyl tRNA in which a second unnatural amino acid is bonded to a tRNA having UUA as an anticodon to pair with a UAA codon and being obtained by modifying a tRNA for tryptophan of Mycoplasma pneumoniae.
<11> An introduction method of an unnatural amino acid, which is a method of introducing at least two types of unnatural amino acids into a polypeptide, the introduction method comprising
   expressing a polypeptide from a nucleic acid by using a cell-free peptide synthesis system which contains
   a nucleic acid having a base sequence including a UAG codon and a UAA codon,
   an aminoacyl tRNA in which a first unnatural amino acid is bonded to a tRNA having CUA as an anticodon to pair with a UAG codon and being obtained by modifying a tRNA for tryptophan of Mycoplasma capricolum, and
   an aminoacyl tRNA in which a second unnatural amino acid is bonded to a tRNA having UUA as an anticodon to pair with a UAA codon and being obtained by modifying a tRNA for tryptophan of Mycoplasma pneumoniae.
<12> The introduction method of an unnatural amino acid according to <11>, in which the base sequence further includes a UGA codon as a stop codon.
<13> A production method of a polypeptide, which is a method of producing a polypeptide in which at least two types of unnatural amino acids are contained in an amino acid sequence, the production method comprising
   expressing a polypeptide from a nucleic acid by using a cell-free peptide synthesis system which contains
   a nucleic acid having a base sequence including a UAG codon and a UAA codon,
   an aminoacyl tRNA in which a first unnatural amino acid is bonded to a tRNA having CUA as an anticodon to pair with a UAG codon and being obtained by modifying a tRNA for tryptophan of Mycoplasma capricolum, and
   an aminoacyl tRNA in which a second unnatural amino acid is bonded to a tRNA having UUA as an anticodon to pair with a UAA codon and being obtained by modifying a tRNA for tryptophan of Mycoplasma pneumoniae.
<14> The production method of a polypeptide according to <13>, in which the base sequence further includes a UGA codon as a stop codon.
<15> A production method of a nucleic acid display library comprising
   expressing a polypeptide from a nucleic acid by using a cell-free peptide synthesis system to produce a nucleic acid-polypeptide conjugate, the cell-free peptide synthesis system containing
   a nucleic acid having a base sequence including a UAG codon and a UAA codon,
   an aminoacyl tRNA in which a first unnatural amino acid is bonded to a tRNA having CUA as an anticodon to pair with a UAG codon and being obtained by modifying a tRNA for tryptophan of Mycoplasma capricolum, and
   an aminoacyl tRNA in which a second unnatural amino acid is bonded to a tRNA having UUA as an anticodon to pair with a UAA codon and being obtained by modifying a tRNA for tryptophan of Mycoplasma pneumoniae.
<16> The production method of a nucleic acid display library according to <15>, in which the production of the nucleic acid-polypeptide conjugate includes producing an mRNA-polypeptide conjugate and reverse-transcribing an mRNA of the mRNA-polypeptide conjugate to produce a cDNA-polypeptide conjugate.
<17> The production method of a nucleic acid display library according to <15> or <16>, in which in the nucleic acid-polypeptide conjugate, the nucleic acid and the polypeptide are linked by a puromycin linker.
<18> A nucleic acid-polypeptide conjugate comprising
   a polypeptide in which at least two types of unnatural amino acids are contained in an amino acid sequence, and
   a nucleic acid having a base sequence encoding the polypeptide, which is linked to the polypeptide,
   in which the base sequence includes a UAG codon and a UAA codon,
   in the base sequence, the UAG codon encodes a first unnatural amino acid in the polypeptide, and
   in the base sequence, the UAA codon encodes a second unnatural amino acid in the polypeptide.
<19> The nucleic acid-polypeptide conjugate according to <18>, in which the nucleic acid-polypeptide conjugate is an mRNA-polypeptide conjugate or a cDNA-polypeptide conjugate.
<20> A screening method comprising
   producing a nucleic acid display library by using the production method of a nucleic acid display library according to any one of <15> to <17>, and
   selecting a nucleic acid-polypeptide conjugate having a target activity from the nucleic acid display library and identifying a base sequence of a nucleic acid of the selected nucleic acid-polypeptide conjugate.

According to the present disclosure, a tRNA which translates a UAA codon into an amino acid, and an aminoacyl tRNA; a reagent for polypeptide synthesis, which introduces at least two types of unnatural amino acids into a polypeptide, an introduction method of an unnatural amino acid, a production method of a polypeptide, and a production method of a nucleic acid display library; a nucleic acid-polypeptide conjugate in which at least two types of unnatural amino acids are introduced into a polypeptide; and a screening method of finding a polypeptide having a target activity from polypeptides into which at least two types of unnatural amino acids are introduced, are provided.

### BRIEF DESCRIPTION OF THE DRAWINGS

Fig. 1 is a diagram showing an example of an embodiment of a tRNA of the present disclosure, and is a schematic diagram of a cloverleaf structure.
Fig. 2 is an electrophoresis gel of tRNA immobilized with formamide.
Fig. 3 is an electrophoresis gel of a polypeptide into which a fluorescent-labeled amino acid and N-methylphenylalanine are multiply introduced.
Fig. 4 is an electrophoresis gel of an mRNA display into which a fluorescent-labeled amino acid and N-methylphenylalanine are multiply introduced.

### DESCRIPTION OF THE PREFERRED EMBODIMENTS

Hereinafter, embodiments according to the present disclosure will be described. These descriptions and Examples are only illustrative of the embodiments and do not limit the ranges of the embodiments. The action mechanism mentioned in the present disclosure includes estimation, and the accuracy thereof does not limit the ranges of the embodiments.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in a numerical range described in the present disclosure, an upper limit value or a lower limit value described in the numerical range may be replaced with a value described in an example.

In the present disclosure, each component may contain a plurality of types of substances corresponding thereto. In the present disclosure, upon referring to an amount of each ingredient in a composition, the amount means a total amount of a plurality of substances present in the composition unless otherwise specified, in a case where a plurality of substances corresponding to each ingredient are present in the composition.

In the present disclosure, a polypeptide refers to a molecule in which amino acids are linked by peptide bonds. The number of amino acid residues of the polypeptide is not limited, and the polypeptide is a term including a protein. It is desirable that the polypeptide in the present disclosure has 6 or more residues of amino acid residues. The polypeptide includes a polypeptide in which an amino acid is post-translationally modified. Examples of the post-translation modification of an amino acid include phosphorylation, methylation, acetylation, and the like.

In the present disclosure, a nucleic acid refers to a molecule carrying information for synthesizing a polypeptide. The nucleic acid is a term including any nucleic acid (for example, DNA, RNA, an analog thereof, a natural product, or an artificial product) and a nucleic acid in which a low-molecular-weight compound, a group, a molecule other than a nucleic acid, a structure, or the like is linked to any nucleic acid. The nucleic acid may be a single-stranded nucleic acid or a double-stranded nucleic acid.

The base sequence of the tRNA specified by SEQ ID NO in the present disclosure is as shown in Table 1.

SEQ ID NO: 2 and SEQ ID NO: 5 to 17 represent variants of a tRNA for tryptophan of Mycoplasma pneumoniae.

SEQ ID NO: 18 represents a wild-type tRNA for tryptophan of Mycoplasma pneumoniae.

SEQ ID NO: 1 and SEQ ID NO: 19 represent variants of a tRNA for tryptophan of Mycoplasma capricolum.

SEQ ID NO: 3 represents a variant of a tRNA for tryptophan of Staphylococcus aureus.

SEQ ID NO: 4 represents a variant of a tRNA for tryptophan of Shewanella oneidensis.

In Table 1, the anticodon is underlined, and bases different from the wild-type tRNA for tryptophan for each microorganism are shown in an italic form.

**[Table 1]**

| SEQ ID NO | Base sequence | Remarks |
|---|---|---|
| SEQ ID NO: 1 | GGGAGAGUAG UUCAAUGGUA GAACGUCGGU CUUUAAAACC GAGCGUUGAG GGUUCGAUUC CUUUCUCUCC CACCA | Mycoplasma capricolum |
| | | G1 U33 U34 C72 A73 |
| | | Codon to be translated is UAA |
| SEQ ID NO: 2 | GGGGGUGUAG UUUAGUGGCA GAACAACAGU CUUUAAAACU GUCUGUGUGG GUUCGAUUCC UUCCACCCCC ACCA | Mycoplasma pneumoniae |
| | | U33 U34 A73 |
| | | Codon to be translated is UAA |
| SEQ ID NO: 3 | GGGGGCAUAG UUCAACGGUA GAAUAGAGGU CUUUAAAACC UUUGGUGUGG GUUCGAUUCC UACUGCCCCC ACCA | Staphylococcus aureus |
| | | G1 U33 U34 C72 A73 |
| | | Codon to be translated is UAA |
| SEQ ID NO: 4 | GGGGGUGUAG UUCCAAUUGG UAGAACAGCG GUCUUUAAAA CCGAUGGUUG CGGGUUCGAG UCCUGCCACC CCCACCA | Shewanella oneidensis |
| | | G1 U33 U34 C72 A73 |
| | | Codon to be translated is UAA |
| SEQ ID NO: 5 | GGCGGUGUAG UUUAGUGGCA GAACAACAGU CUUUAAAACU GUCUGUGUGG GUUCGAUUCC UUCCACCGCC ACCA | Mycoplasma pneumoniae |
| | | C3 U33 U34 G70 A73 |
| | | Codon to be translated is UAA |
| SEQ ID NO: 6 | GGAGGUGUAG UUUAGUGGCA GAACAACAGU CUUUAAAACU GUCUGUGUGG GUUCGAUUCC UUCCACCUCC ACCA | Mycoplasma pneumoniae |
| | | A3 U33 U34 U70 A73 |
| | | Codon to be translated is UAA |
| SEQ ID NO: 7 | GGUGGUGUAG UUUAGUGGCA GAACAACAGU CUUUAAAACU GUCUGUGUGG GUUCGAUUCC UUCCACCACC ACCA | Mycoplasma pneumoniae |
| | | U3 U33 U34 A70 A73 |
| | | Codon to be translated is UAA |
| SEQ ID NO: 8 | GGGGGUGUAG UUUAGUGGCA GAACAACAGU CUUUAAAACU GUCUGUGUGG GUUCGAUUCC UUCCACCUCC ACCA | Mycoplasma pneumoniae |
| | | G3 U33 U34 U70 A73 |
| | | Codon to be translated is UAA |
| SEQ ID NO: 9 | GGUGGUGUAG UUUAGUGGCA GAACAACAGU CUUUAAAACU GUCUGUGUGG GUUCGAUUCC UUCCACCGCC ACCA | Mycoplasma pneumoniae |
| | | U3 U33 U34 G70 A73 |
| | | Codon to be translated is UAA |
| SEQ ID NO: 10 | GGGCGUGUAG UUUAGUGGCA GAACAACAGU CUUUAAAACU GUCUGUGUGG GUUCGAUUCC UUCCACGCCC ACCA | Mycoplasma pneumoniae |
| | | C4 U33 U34 G69 A73 |
| | | Codon to be translated is UAA |
| SEQ ID NO: 11 | GGGAGUGUAG UUUAGUGGCA GAACAACAGU CUUUAAAACU GUCUGUGUGG GUUCGAUUCC UUCCACUCCC ACCA | Mycoplasma pneumoniae |
| | | A4 U33 U34 U69 A73 |
| | | Codon to be translated is UAA |
| SEQ ID NO: 12 | GGGUGUGUAG UUUAGUGGCA GAACAACAGU CUUUAAAACU GUCUGUGUGG GUUCGAUUCC UUCCACACCC ACCA | Mycoplasma pneumoniae |
| | | U4 U33 U34 A69 A73 |
| | | Codon to be translated is UAA |
| SEQ ID NO: 13 | GGGGGUGUAG UUUAGUGGCA GAACAACAGU CUUUAAAACU GUCUGUGUGG GUUCGAUUCC UUCCACUCCC ACCA | Mycoplasma pneumoniae |
| | | G4 U33 U34 U69 A73 |
| | | Codon to be translated is UAA |
| SEQ ID NO: 14 | GGGUGUGUAG UUUAGUGGCA GAACAACAGU CUUUAAAACU GUCUGUGUGG GUUCGAUUCC UUCCACGCCC ACCA | Mycoplasma pneumoniae |
| | | U4 U33 U34 G69 A73 |
| | | Codon to be translated is UAA |
| SEQ ID NO: 15 | GGGGGUGUAG UUUAGUGGCA GAACAACAGU CUUUAAAACU GUCUGUGUGG GUUCGAUUCC UUCCACCCCC GCCA | Mycoplasma pneumoniae |
| | | U33 U34 G73 |
| | | Codon to be translated is UAA |
| SEQ ID NO: 16 | GGUGGUGUAG UUUAGUGGCA GAACAACAGU CUUUAAAACU GUCUGUGUGG GUUCGAUUCC UUCCACCGCC GCCA | Mycoplasma pneumoniae |
| | | U3 U33 U34 G70 G73 |
| | | Codon to be translated is UAA |
| SEQ ID NO: 17 | GGGGGUGUAG UUUAGUGGCA GAACAACAGU CUUUAAAACU GUCUGUGUGG GUUCGAUUCC UUCCACUCCC GCCA | Mycoplasma pneumoniae |
| | | G4 U33 U34 U69 G73 |
| | | Codon to be translated is UAA |
| SEQ ID NO: 18 | GGGGGUGUAG UUUAGUGGCA GAACAACAGU CUCCAAAACU GUCUGUGUGG GUUCGAUUCC UUCCACCCCC GCCA | Mycoplasma pneumoniae |
| | | Wild-type |
| | | Codon to be translated is UGG |
| SEQ ID NO: 19 | GGGAGAGUAG UUCAAUGGUA GAACGUCGGU CUCUAAAACC GAGCGUUGAG GGUUCGAUUC CUUUCUCUCC CACCA | Mycoplasma capricolum |
| | | G1 U34 C72 A73 |
| | | Codon to be translated is UAG |

The tRNA found in nature has a range of the number of bases from 70 to 90. A typical tRNA has the number of bases of 76. The base position described in the remarks column of Table 1 is a position specified in the comparison with a typical tRNA having the number of bases of 76. Taking SEQ ID NO: 2 as an example, although the number of bases is 74, the position of the 3' terminal is specified as a base number 76, and the position adjacent to the 5' side of the CCA terminal is specified as a base number 73.

### <tRNA>

The tRNA of the present disclosure is a tRNA obtained by modifying a tRNA for tryptophan of Mycoplasma pneumoniae, the tRNA including a UUA as an anticodon to pair with a UAA codon. The tRNA of the present disclosure translates the UAA codon into an amino acid.

Mycoplasma pneumoniae has two types of wild-type tRNAs for tryptophan (Nucleic Acids Res. 1993 Oct 25; 21 (21): 4967-4974). The tRNA of the present disclosure includes any of the variants of the two types of wild-type tRNAs for tryptophan. The tRNA of the present disclosure is preferably a tRNA obtained by modifying the wild-type tRNA for the tryptophan represented by SEQ ID NO: 18.

The tRNA of the present disclosure is a tRNA in which an anticodon of wild-type tRNA for the tryptophan of Mycoplasma pneumoniae is modified from CCA to UUA. Because of this modification, the tRNA of the present disclosure has a function of translating the UAA codon into an amino acid.

As a result of modifying and examining wild-type tRNAs for tryptophan of various microorganisms, the inventors of the present disclosure have found that a variant of tRNA for tryptophan of Mycoplasma pneumoniae efficiently translates a UAA codon into an amino acid.

The tRNA of the present disclosure is preferably a variant of wild-type tRNA for the tryptophan of Mycoplasma pneumoniae in which not only the anticodon but also an acceptor stem is modified. It is presumed that the modification of the acceptor stem affects the higher-order structure of tRNA and increases the efficiency of the peptidyl transfer reaction in the ribosome. Examples of the modification of the acceptor stem include the modification of the base adjacent to the 5' side of the CCA terminal; the modification of the combination of the base at the third position from the 5' terminal and a base paired with the base; and the modification of the combination of the base at the fourth position from the 5' terminal and a base paired with the base.

The CCA terminal of the tRNA is three bases that are universally present at the 3' terminal of the tRNA.

The pairing within the tRNA means that the base pairs face each other in the cloverleaf structure of the tRNA.

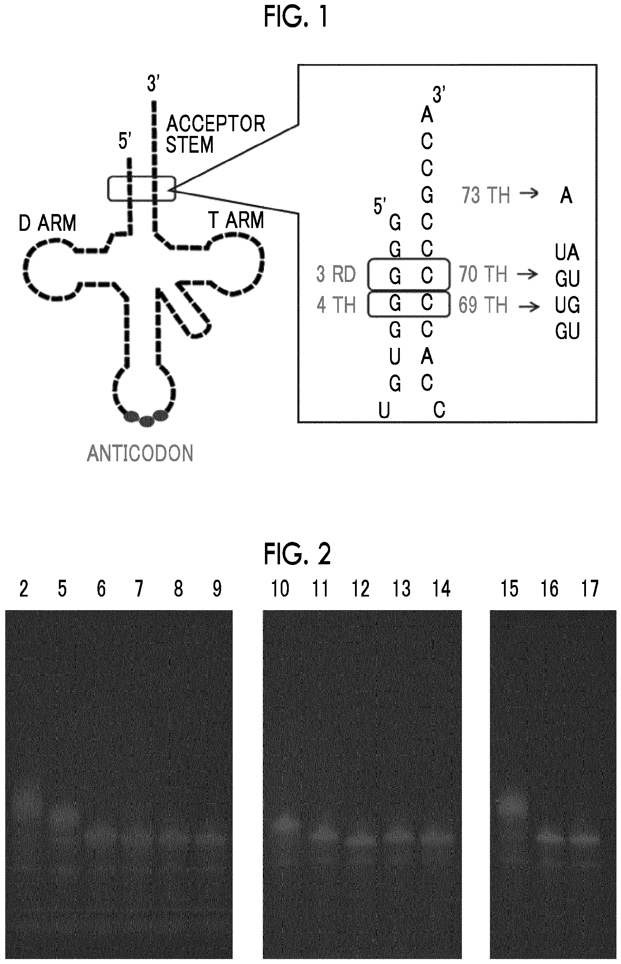

Fig. 1 is a diagram showing an example of an embodiment of a tRNA of the present disclosure, and is a schematic diagram of a cloverleaf structure. Fig. 1 shows the modification example of the base (base number 73) adjacent to the 5' side of the CCA terminal; the modification example of the combination of the base at the third position from the 5' terminal and a base (base number 70) paired with the base; and the modification of the combination of the base at the fourth position from the 5' terminal and a base (base number 69) paired with the base.

From the viewpoint of efficiently translating the UAA codon into an amino acid, the tRNA of the present disclosure preferably has at least one form selected from the group consisting of Form (1), Form (2), and Form (3).

Form (1): Abase adjacent to a 5' side of the CCA terminal is A or G.

Form (2): the combination of a base at a third position from a 5' terminal and a base paired with the base is UA, GU, or UG. Here, in the description of two bases, the third base from the 5' terminal is described first. For example, in "UA", the third base from the 5' terminal is U, and the base paired with the third base in the cloverleaf structure is A.

Form (3): the combination of a base at a fourth position from a 5' terminal and a base paired with the base is GU. Here, in the description of two bases, the fourth base from the 5' terminal is described first. The fourth base from the 5' terminal is G, and a base paired with the fourth base in the cloverleaf structure is U.

Examples of embodiments of the tRNA of the present disclosure include a tRNA having Form (1) and Form (2) and a tRNA having Form (1) and Form (3).

Examples of embodiment of the tRNA of the present disclosure include a tRNA having the base sequence set forth in SEQ ID NO: 2, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, or SEQ ID NO: 17. Among these, from the viewpoint of efficiently translating the UAA codon into an amino acid, the tRNA having the base sequence set forth in SEQ ID NO: 7, SEQ ID NO: 8, SEQ ID NO: 9, SEQ ID NO: 13, SEQ ID NO: 15, or SEQ ID NO: 17 is preferable.

The tRNA of the present disclosure can be produced by using a known genetic engineering technique based on the base sequence of the wild-type tRNA for tryptophan of Mycoplasma pneumoniae. For example, a tRNA gene is designed, a tRNA gene is produced by polymerase chain reaction (PCR) using an appropriate primer, and the tRNA is transcribed from the tRNA gene.

### <Aminoacyl tRNA>

The aminoacyl tRNA of the present disclosure is a tRNA in which an amino acid is bonded to the tRNA of the present disclosure. In the aminoacyl tRNA, the amino acid is covalently bonded to the 3' terminal of the tRNA.

The tRNA of the present disclosure can be acylated with any amino acid. Therefore, the type of amino acid contained in the aminoacyl tRNA of the present disclosure is not limited. Here, the amino acid includes a natural amino acid, an unnatural amino acid, a modified amino acid, and a derivative thereof.

The natural amino acid in the present disclosure refers to an amino acid constituting a common protein, that is alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. The natural amino acid may be a natural product or an artificial product.

In the present disclosure, the unnatural amino acid refers to an amino acid other than the twenty types of amino acids described above, and includes a natural product or an artificial product.

An example of the unnatural amino acid is an amino acid having a chloroacetyl group (for example, a chloroacetylated amino acid such as a chloroacetyl lysine, a chloroacetyl diaminobutyric acid, and the like).

An example of the unnatural amino acid is an N-methylamino acid (for example, N-methylalanine and N-methylphenylalanine).

An example of the modified amino acid is a labeled amino acid which is an amino acid bonded to a labeling compound. The labeling compound is a substance that can be detected by a biochemical, chemical, immunochemical, or electromagnetic detection method. Examples of the labeling compound include a coloring agent compound, a fluorescent substance, a chemiluminescent substance, a bioluminescent substance, an enzyme substrate, a coenzyme, an antigenic substance, a substance that binds to a specific protein, and a magnetic substance. The labeled amino acid is, as classified according to function, for example, a fluorescent-labeled amino acid, a photoresponsive amino acid, a photoswitch amino acid, a fluorescent probe amino acid, and the like.

In the labeled amino acid, the amino acid and the labeling compound may be directly bonded to each other, or the amino acid and the labeling compound may be bonded through a spacer. Examples of the spacer include polyolefins such as polyethylene and polypropylene; polyethers such as polyoxyethylene, polyethylene glycol, and polyvinyl alcohol; polystyrene, polyvinyl chloride, polyester, polyamide, polyimide, polyurethane, polycarbonate, and the like.

Examples of the derivative of the natural amino acid, the unnatural amino acid, or the modified amino acid include hydroxy acids, mercapto acids, and carboxylic acids.

Examples of embodiments of the aminoacyl tRNA of the present disclosure include an aminoacyl tRNA in which one selected from the group consisting of chloroacetyl lysine, N-methylalanine, N-methylphenylalanine, and a fluorescent-labeled amino acid is bonded to tRNA.

Examples of a method of acylating the tRNA of the present disclosure with an amino acid include the following method.

The method includes omitting the CA dinucleotide at the 3' terminal of the tRNA, on the other hand, bonding a CA dinucleotide to the carboxy group of the amino acid, and linking both of these by using an RNA ligase. The present method is known, and the details thereof are disclosed in, for example, WO2004/009709A and WO2007/055429A. The methods disclosed in these publications can be adopted for producing the aminoacyl tRNA of the present disclosure.

### <Reagent for polypeptide synthesis>

The present disclosure provides two forms of the reagent for polypeptide synthesis (that is, a first reagent for polypeptide synthesis and a second reagent for polypeptide synthesis).

The first reagent for polypeptide synthesis contains
a tRNA having CUA as an anticodon to pair with a UAG codon, the tRNA being obtained by modifying a tRNA for tryptophan of Mycoplasma capricolum, and
a tRNA having UUA as an anticodon to pair with a UAA, the tRNA being obtained by modifying a tRNA for tryptophan of Mycoplasma pneumoniae codon.

In the first reagent for polypeptide synthesis, the above-described two types of tRNAs may be in a mixed state or in a non-mixed state. The first reagent for polypeptide synthesis may further contain a tRNA other than the above-described two types of tRNAs.

The second reagent for polypeptide synthesis contains
an aminoacyl tRNA in which a first unnatural amino acid is bonded to a tRNA having CUA as an anticodon to pair with a UAG codon and being obtained by modifying a tRNA for tryptophan of Mycoplasma capricolum, and
an aminoacyl tRNA in which a second unnatural amino acid is bonded to a tRNA having UUA as an anticodon to pair with a UAA codon and being obtained by modifying a tRNA for tryptophan of Mycoplasma pneumoniae.

In the second reagent for polypeptide synthesis, the above-described two types of aminoacyl tRNAs may be in a mixed state or in a non-mixed state. The second reagent for polypeptide synthesis may further contain an aminoacyl tRNA other than the above-described two types of aminoacyl tRNAs.

In the second reagent for polypeptide synthesis, the first unnatural amino acid and the second unnatural amino acid are different types of amino acids. Each of the first unnatural amino acid and the second unnatural amino acid includes an unnatural amino acid; a modified amino acid of a natural amino acid or an unnatural amino acid; or a derivative of a natural amino acid, an unnatural amino acid, or a modified amino acid. Details of these amino acids are as described above.

Hereinafter, a tRNA which is obtained by modifying a tRNA for tryptophan of Mycoplasma capricolum and which has CUA as an anticodon to pair with a UAG codon is referred to as "UAG codon-translating tRNA".

Hereinafter, a tRNA which is obtained by modifying a tRNA for tryptophan of Mycoplasma pneumoniae and which has UUA as an anticodon to pair with a UAA codon is referred to as "UAA codon-translating tRNA".

The reagent for polypeptide synthesis of the present disclosure is a reagent that uses a UAG codon-translating tRNA and a UAA codon-translating tRNA in combination. In a case where both tRNAs are acylated with separate unnatural amino acids and used in combination, at least two types of unnatural amino acids can be introduced into the polypeptide.

The first reagent for polypeptide synthesis is used, for example, by being acylated with an unnatural amino acid and then added to a cell-free peptide synthesis system. The second reagent for polypeptide synthesis is used, for example, by being added to a cell-free peptide synthesis system. According to these using forms, at least two types of unnatural amino acids can be introduced into the polypeptide synthesized by the cell-free peptide synthesis system. Details of the cell-free peptide synthesis system are as described later.

It is sufficient that the first reagent for polypeptide synthesis includes at least a UAG codon-translating tRNA and a UAA codon-translating tRNA among elements required for polypeptide synthesis.

It is sufficient that the second reagent for polypeptide synthesis includes at least a UAG codon-translating tRNA to which the first unnatural amino acid is bonded and a UAA codon-translating tRNA to which the second unnatural amino acid is bonded among elements required for polypeptide synthesis.

The reagent for polypeptide synthesis of the present disclosure may contain all the elements required for polypeptide synthesis. The reagent for polypeptide synthesis of the present disclosure may include an instrument used for polypeptide synthesis.

A UAG codon-translating tRNA which is a variant of the tRNA for tryptophan of Mycoplasma capricolum is disclosed in WO2007/055429A. The tRNA variant disclosed in WO2007/055429A can be adopted in the reagent for polypeptide synthesis of the present disclosure, and an introduction method of an unnatural amino acid, a production method of a polypeptide, and a production method of a nucleic acid display library, which are described later.

From the viewpoint of efficiently translating UAG codon into an amino acid, the UAG codon-translating tRNA, which is a variant of the tRNA for tryptophan of Mycoplasma capricolum, preferably includes at least one form selected from the group consisting of Form (A), Form (B), and Form (C).

Form (A): the combination of the base of the 5' terminal and a base paired with the base is GC. Here, in the description of two bases, the base of the 5' terminal is described first. The base of the 5' terminal is G, and a base paired with the base of the 5' terminal in the cloverleaf structure is C.

Form (B): the fourth base from the 3' terminal is A.

Form (C): A, C, G, or U is inserted at a position adjacent to the 5' side of the CCA terminal.

A variant of tRNA for tryptophan of Mycoplasma capricolum can be produced by using a known genetic engineering technique based on the base sequence of the wild-type tRNA for tryptophan of Mycoplasma capricolum. For example, a tRNA gene is designed, a tRNA gene is produced by polymerase chain reaction (PCR) using an appropriate primer, and the tRNA is transcribed from the tRNA gene.

### introduction method of unnatural amino acid and production method of polypeptide>

The introduction method of an unnatural amino acid of the present disclosure is a method of introducing at least two types of the unnatural amino acids into a polypeptide, and includes expressing the polypeptide from a nucleic acid by a cell-free peptide synthesis system.

A production method of a polypeptide of the present disclosure is a method for producing a polypeptide in which at least two types of unnatural amino acids are contained in an amino acid sequence, and includes expressing a polypeptide from a nucleic acid by a cell-free peptide synthesis system.

The cell-free peptide synthesis system in the introduction method of an unnatural amino acid of the present disclosure and the production method of a polypeptide of the present disclosure contains,
a nucleic acid having a base sequence including a UAG codon and a UAA codon,
an aminoacyl tRNA in which a first unnatural amino acid is bonded to a UAG codon-translating tRNA which is a variant of a tRNA for tryptophan of Mycoplasma capricolum, and
an aminoacyl tRNA in which a second unnatural amino acid is bonded to a UAA codon-translating tRNA which is a variant of a tRNA for tryptophan of Mycoplasma pneumoniae.

The first unnatural amino acid and the second unnatural amino acid are different types of amino acids. Each of the first unnatural amino acid and the second unnatural amino acid includes an unnatural amino acid; a modified amino acid of a natural amino acid or an unnatural amino acid; or a derivative of a natural amino acid, an unnatural amino acid, or a modified amino acid. Details of these amino acids are as described above.

The cell-free peptide synthesis system is a reaction system for polypeptide synthesis, and is, without using cells themselves, (1) a system configured to perform translation of a nucleic acid, or (2) a system configured to perform transcription and translation of a nucleic acid. The cell-free peptide synthesis system is composed of a template nucleic acid, a ribosome, a factor and an enzyme for transcription and/or translation, enzymes that are necessary for the constitution of the system, various substrates, an energy source, a buffer solution, and salts. Examples of the factor and enzyme for transcription and/or translation include a substance derived from a prokaryotic cell such as Escherichia coli; a substance derived from a eukaryotic cell such as wheat germ, an animal cell, or an insect cell; and the like.

In the cell-free peptide synthesis system, the template nucleic acid may be DNA or RNA. In the DNA, the UAG codon is read as TAG, and the UAA codon is read as TAA.

The template nucleic acid may be a single-stranded nucleic acid or a double-stranded nucleic acid. The template nucleic acid may be a linear nucleic acid or a circular nucleic acid. The template nucleic acid may be a nucleic acid in which a base sequence required for polypeptide synthesis is incorporated into a vector (a plasmid vector, a cosmid vector, or the like).

The template nucleic acid has a base sequence required to synthesize a polypeptide by a cell-free peptide synthesis system. The base sequence required for polypeptide synthesis is not only a base sequence (that is, a coding region) in which codons corresponding to the amino acid sequence of the polypeptide are arranged, but also for example, a promoter sequence and a ribosome binding sequence.

The base sequence of the template nucleic acid may include a stop codon or may not include a stop codon. The stop codon means a codon that has no pairing with tRNA. An example of the stop codon is a UGA codon. In a case where the base sequence of the template nucleic acid does not contain a stop codon, an mRNA-ribosome-polypeptide conjugate can be formed.
(1) A cell-free peptide synthesis system that performs translation of a nucleic acid (for example, RNA) contains a ribosome, a translation initiation factor, a translation elongation factor, a translation termination factor, an aminoacyl-tRNA synthase, a tRNA acylated by an aminoacyl-tRNA synthase, and the tRNA of the present disclosure bonded to an amino acid, and in a case of a system derived from Escherichia coli, further contains methionyl tRNA transformylase.
(2) A cell-free peptide synthesis system that performs transcription and translation of a nucleic acid (for example, DNA) contains, in addition to the constituent substances of (1), an RNA polymerase (for example, T7 RNA polymerase) and a nucleoside triphosphate that is a substrate of RNA polymerase.

In both (1) and (2), the mRNA-ribosome-polypeptide conjugate can be formed by removing the translation termination factor from the constituent substances.

Examples of the enzymes other than the factor and enzyme for transcription and/or translation include enzymes for regeneration of energy such as creatine kinase, myokinase, and nucleoside diphosphate kinase (NDPK); enzymes for decomposition of inorganic pyrophosphate generated by transcription and/or translation of inorganic pyrophosphatase or the like; and the like.

Examples of various substrates include a natural amino acid and/or an unnatural amino acid, and a nucleotide triphosphate, a creatine phosphate, a formylfolic acid as an energy source. Examples of the nucleotide triphosphate include ATP, GTP, CTP, and UTP. ATP and GTP are used in (1), and ATP, GTP, CTP, and UTP are used in (2).

As the buffer solution, a potassium phosphate buffer solution (pH 7.3) is commonly used. As the salts, potassium glutamate, ammonium chloride, magnesium acetate, calcium chloride, putrescine, spermidine, dithiothreitol (DTT), or the like is commonly used.

In the introduction method of an unnatural amino acid of the present disclosure and the production method of a polypeptide of the present disclosure, any known cell-free peptide synthesis system can be adopted. Examples of the commercially available product of the cell-free peptide synthesis system include PUREfrex (GeneFrontier, "PUREfrex" is a registered trademark), Human Cell-Free Protein Expression System (Takara Bio), Rapid Translation System (Roche), Expressway Cell-Free Expression System (Invitrogen).

In the introduction method of an unnatural amino acid of the present disclosure and the production method of a polypeptide of the present disclosure, the UAG codon and the UAA codon of the base sequence encoding the polypeptide are translated by UAG codon-translating tRNA to which the first unnatural amino acid is bonded and UAA codon-translating tRNA to which the second unnatural amino acid is bonded, respectively, and at least two types of the unnatural amino acids are introduced into the polypeptide.

Examples of the embodiment of the polypeptide include a polypeptide containing an N-methylamino acid (for example, N-methylalanine or N-methylphenylalanine), an amino acid having a chloroacetyl group (for example, chloroacetyl diaminobutyric acid or chloroacetyl lysine), and an amino acid having a thiol group (for example, cysteine) in one molecule. For the synthesis of this polypeptide, a UAG codon-translating tRNA to which N-methylamino acid is bonded and a UAA codon-translating tRNA to which an amino acid having chloroacetyl group is bonded (or a UAG codon-translating tRNA to which an amino acid having chloroacetyl group is bonded and a UAA codon-translating tRNA to which N-methylamino acid is bonded) may be used. In this polypeptide, the chloroacetyl group and the thiol group react with each other to be cyclized, resulting in a cyclic N-methyl polypeptide. The number of amino acid residues of the cyclic N-methyl polypeptide is, for example, 3 to 20.

### <Production method of nucleic acid display library and nucleic acid-polypeptide conjugate>

The nucleic acid display library has a nucleic acid-polypeptide conjugate as a constitutional unit, and indicates a collection of a plurality of nucleic acid-polypeptide conjugates. The number of clones and the number of copies of the nucleic acid-polypeptide conjugate constituting the nucleic acid display library are not limited.

The nucleic acid-polypeptide conjugate of the present disclosure is a nucleic acid-polypeptide conjugate containing a polypeptide in which at least two types of unnatural amino acids are contained in an amino acid sequence, and a nucleic acid having a base sequence encoding the polypeptide, which is linked to the polypeptide,
in which the base sequence includes a UAG codon and a UAA codon,
in the base sequence, the UAG codon encodes the first unnatural amino acid in the polypeptide, and
in the base sequence, the UAA codon encodes the second unnatural amino acid in the polypeptide.

Examples of the embodiment of the nucleic acid-polypeptide conjugate of the present disclosure include an mRNA-polypeptide conjugate or a cDNA-polypeptide conjugate. The cDNA-polypeptide conjugate is a reverse transcription product of the mRNA-polypeptide conjugate. In the cDNA-polypeptide conjugate, the UAG codon is read as TAG, and the UAA codon is read as TAA.

In the nucleic acid-polypeptide conjugate of the present disclosure, the nucleic acid and the polypeptide are linked, for example, through a puromycin linker or a ribosome.

A production method of a nucleic acid display library of the present disclosure includes expressing a polypeptide from a nucleic acid by a cell-free peptide synthesis system to produce a nucleic acid-polypeptide conjugate.

The cell-free peptide synthesis system in the production method of a nucleic acid display library of the present disclosure contains, for the purpose of introducing at least two types of unnatural amino acids into a polypeptide,
a nucleic acid having a base sequence including a UAG codon and a UAA codon,
an aminoacyl tRNA in which a first unnatural amino acid is bonded to a UAG codon-translating tRNA which is a variant of a tRNA for tryptophan of Mycoplasma capricolum, and
an aminoacyl tRNA in which a second unnatural amino acid is bonded to a UAA codon-translating tRNA which is a variant of a tRNA for tryptophan of Mycoplasma pneumoniae.

The cell-free peptide synthesis system may be any of (1) a system configured to perform translation of a nucleic acid or (2) a system configured to perform transcription and translation of a nucleic acid. Details of the cell-free peptide synthesis system are as described above. In the production method of a nucleic acid display library of the present disclosure, any known cell-free peptide synthesis system and any known nucleic acid display library production technique can be adopted.

In the cell-free peptide synthesis system, the template nucleic acid may be DNA or RNA. The template nucleic acid is, for example, a population of double-stranded DNA fragments produced by performing overlap extension PCR using a random primer set including a random sequence. The random sequence is, for example, a repeating sequence [NNK]m of triplet (here, m is a positive integer, N's each independently represent A, T, G, or C, and K each independently represents T or G). By setting the number of repetitions of the triplet [NNK] to any number, a peptide having any length can be produced. From the viewpoint of suppressing the appearance of stop codon, the random sequence is preferably a trimer oligonucleotide in which one type of codon is assigned to one type of amino acid.

From the viewpoint of increasing the flexibility of the polypeptide, a base sequence encoding a spacer is preferably present on the 3' terminus side of the template nucleic acid. The spacer is, for example, at least one amino acid selected from glycine or serine.

As an example of the embodiment of the production method of a nucleic acid display library of the present disclosure, the method includes producing an mRNA-polypeptide conjugate, and producing a cDNA-polypeptide conjugate by reverse transcription of mRNA of the mRNA-polypeptide conjugate. According to the examples of the present embodiment, since the nucleic acid of the nucleic acid-polypeptide conjugate is DNA, a chemically more stable nucleic acid display library can be obtained.

In the production method of a nucleic acid display library of the present disclosure, linking between a nucleic acid to be translated (usually mRNA) and a translation product may be any one of linking through a puromycin linker, linking through a ribosome, or the like. From the viewpoint that the translation product easily forms an appropriate higher-order structure and from the viewpoint that the function of the translation product is easily evaluated, the linking between the nucleic acid to be translated and the translation product is preferably a linking through a puromycin linker.

Accordingly, the nucleic acid to be translated in the cell-free peptide synthesis system is preferably a nucleic acid in which a puromycin linker is added to the 3' terminal. From the viewpoint of suppressing the dissociation of the nucleic acid and puromycin, the linker for adding puromycin to a nucleic acid is preferably a 2'-O-methylated nucleic acid linker or a nucleic acid linker having an ultraviolet crosslinking compound at the 5' terminal.

In the production method of a nucleic acid display library of the present disclosure, since a UAG codon and a UAA codon are used for translation of an amino acid while the codons of twenty types of natural amino acids are stored, the amino acid sequence of the polypeptide constituting the nucleic acid display library can be diversified.

### <Screening method>

A screening method of the present disclosure includes producing a nucleic acid display library by the production method of a nucleic acid display library of the present disclosure, and selecting a nucleic acid-polypeptide conjugate having a target activity from the nucleic acid display library and identifying a base sequence of a nucleic acid of the selected nucleic acid-polypeptide conjugate.

The "target activity" is, for example, binding to a target substance. The target substance is a term including any chemical substance exhibiting physiological activity, and is a term including a compound, a group, a molecule, a protein, a nucleic acid, a lipid, a saccharide, a complex of these, and the like. The target substance is, for example, a receptor, a transcription factor, an enzyme, a coenzyme, a regulating factor, an antibody, an antigen, DNA, RNA, a fragment thereof, a composite body thereof, and a modification group thereof.

An example of the embodiment of the screening method of the present disclosure includes bringing a nucleic acid display library into contact with a target substance (for example, a target protein) and performing incubation. For example, the nucleic acid display library is brought into contact with the target substance in a buffer solution, and incubated with adjusted pH and temperature of the buffer solution. In this case, a target substance may be immobilized on a solid phase carrier, and then a nucleic acid display library may be brought into contact with the immobilized target substance. The solid phase carrier is not limited as long as it is a carrier on which a target substance can be immobilized, and examples thereof include a microtiter plate, a substrate, a bead, a magnetic bead, a nitrocellulose membrane, a nylon membrane, a PVDF membrane, and the like. The target substance is immobilized on these solid phase carriers by a known technique.

After the contact described above, the nucleic acid-polypeptide conjugate bound to a target substance (for example, a target protein) is extracted, and the base sequence of the nucleic acid of the extracted nucleic acid-polypeptide conjugate is identified. Identification of the base sequence can be carried out using a nucleic acid amplification system and a sequencer.

The nucleic acid amplification system refers to a system that amplifies a nucleic acid using a nucleic acid as a template. The nucleic acid amplification reaction of the nucleic acid amplification system can be any one of polymerase chain reaction (PCR), ligase chain reaction (LCR), transcription mediated amplification (TMA), nucleic acid sequence-based amplification (NASBA), or the like.

In the present disclosure, the sequencer is a term including a first generation sequencer (a capillary sequencer), a second generation sequencer (a next generation sequencer), a third generation sequencer, a fourth generation sequencer, and a sequencer to be developed in the future. The sequencer may be a capillary sequencer, may be a next generation sequencer, or may be another sequencer. The sequencer is preferably a next generation sequencer from the viewpoints of the speed of analysis, the large number of specimens that can be processed at one time, and the like. The next generation sequencer (NGS) refers to a sequencer that is classified by being contrasted with a capillary sequencer (called a first generation sequencer) using the Sanger method. At present, the most popular next generation sequencer is a sequencer of which the principle is to capture fluorescence or luminescence linked to a complementary strand synthesis by DNA polymerase or a complementary strand binding by DNA ligase and determine the base sequence. Specific examples thereof include MiSeq (Illumina, Inc., MiSeq is a registered trademark), HiSeq 2000 (Illumina, Inc., HiSeq is a registered trademark), and Roche 454 (Roche, Ltd.).

According to the screening method of the present disclosure, a polypeptide having a target activity can be found from polypeptides into which at least two types of unnatural amino acids are introduced, and a base sequence encoding the polypeptide and an amino acid sequence thereof can be found.

### Examples

The tRNA and the like of the present disclosure will be described in more detail below with reference to specific examples. The materials, treatment procedures, and the like shown in the specific examples below can be changed as appropriate without departing from the gist of the present disclosure. The scope of the tRNA and the like of the present disclosure should not be construed as being limited by the following specific examples.

In the following description, unless otherwise specified, the synthesis, treatment, manufacture, and the like were performed at room temperature (25°C ± 3°C). The percentage related to the substance concentration is based on mass. "M" related to the substance concentration represents a molar concentration, and 1 M = 1 mol/L.

In the following description, the abbreviations have the following meanings.
AcOK: potassium acetate
α-CHCA: α-cyano-4-hydroxycinnamic acid
BSA: bovine serum albumin
DMSO: dimethyl sulfoxide
dNTP: deoxynucleoside triphosphate
D-PBS: Dulbecco's phosphate buffered saline
DTT: dithiothreitol
GMP: guanosine monophosphate
NTP: nucleoside triphosphate
SDS-PAGE: sodium dodecyl sulphate-polyacrylamide gel electrophoresis

### <Example 1>

Respective tRNA for tryptophan of Mycoplasma capricolum, Mycoplasma pneumoniae, Staphylococcus aureus, and Shewanella oneidensis were modified, and tRNAs of SEQ ID NOs: 1 to 4 were designed respectively as tRNA having UUA in the anticodon.

### -Production of tRNA gene-

Two oligonucleotides were chemically synthesized as primer based on the base sequences set forth in SEQ ID NOs: 1 to 4. The forward primer contains a T7 promoter and the base sequence of positions 1 to 51 of the tRNA. The reverse primer contains the base sequence of positions 36 to 74 (up to the first C of the CCA terminal) of the tRNA. PCR was performed using these primers, and the PCR product was purified to obtain a tRNA gene. This gene is referred to as a tRNA-CA gene. The PCR reaction solution contains a KOD Dash Buffer, 0.2 mM dNTP, 2.5 units of KOD Dash, and 0.2 nmol of the respective primers are contained in 100 µL. A MinElute PCR Purification Kit (QIAGEN N.V.) was used for purifying a PCR product.

### -Production of tRNA-

The tRNA-CA gene was transcribed, and the transcription product was purified to obtain tRNA. This tRNA is referred to as tRNA-CA. 100 µL of the reaction solution of transcription contains 40 mM Tris-HCl (pH 8.0), 20 mM MgCl₂, 5 mM DTT, 4 mM NTP, 20 mM GMP, 2 mM Spermidine, 10 µg/mL BSA, 40 units of Ribonuclease inhibitor, 0.5 unit of Inorganic Pyrophosphatase, 400 units of T7 RNA Polymerase, and 6 µg of tRNA-CA gene. The reaction solution was reacted at 37°C for 12 hours, and tRNA-CA was purified using an RNeasy Minelute Cleanup Kit (QIAGEN N.V).

### -Aminoacylation of tRNA-

BODIPYFL-aminophenylalanine was boneded to tRNA. This tRNA is referred to as a fluorescent-labeled amino acid-tRNA. 4 µL of 5× Ligation Buffer (275 mM Hepes-Na (pH 7.5), 75 mM MgCl₂, 16.5 mM DTT, and 5 mM ATP), 2.5 µL of 200 µM tRNA-CA, 2 µL of DMSO solution of BODIPYFL-aminophenylalanine-pdCpA, 0.4 µL of 0.1% BSA, 0.6 µL of T4 RNALigase (40 units/µL), and 10.5 µL of water were mixed and reacted at 4°C for 2 hours. 20 µL of 0.3 M AcOK (pH 4.5) and 120 µL of ethanol were added thereto, mixed gently, and allowed to stand at -80°C for 30 minutes. After centrifugation at 15,000 rpm for 30 minutes at 4°C, the supernatant was removed, 200 µL of 70% ethanol stored at -30°C was added thereto, and the mixture was centrifuged at 15,000 rpm for 1 minute at 4°C. The supernatant was removed, followed by drying under reduced pressure. The dried resultant was dissolved in 3 µL of 1 mM AcOK (pH 4.5).

### -Introduction of fluorescent-labeled amino acid into polypeptide-

A DNA containing a base sequence (SEQ ID NO: 21: ATGTGCAAATAAAAACCGCGGAGCAAAAACATGAGCGATTATAAAGATGATGATGA TAAG) encoding a polypeptide consisting of twenty amino acids (SEQ ID NO: 20: MCKXKPRSKNMSDYKDDDDK, X is an unnatural amino acid) was prepared. This polypeptide and DNA are referred to as a Polypeptide (1) and DNA (1). Polypeptide (1) contains a FLAG tag, and positions 13 to 20 of the amino acid sequences is the FLAG tag. DNA (1) includes all base sequences required for the expression of Polypeptide (1) by PUREfrex 2.0.

DNA (1) was transcribed, and the transcription product was purified to obtain mRNA. The transcription from DNA (1) was performed in the same manner as the transcription from the tRNA-CA gene, except that 20 mM GMP was not added to the reaction solution.

Polypeptide (1) was translated from mRNA, which is a transcription product and a purification product of DNA (1), using fluorescent-labeled amino acid-tRNA and PUREfrex 2.0 (GeneFrontier Corporation). 10 µL of the reaction solution includes 5 µL of Solution I of PUREfrex 2.0, 0.5 µL of Solution II, 1 µL of Solution III, 1 µL of 16 µM mRNA, 1 µL of a fluorescent-labeled amino acid-tRNA solution, and 1.5 µL of water. The translation reaction was performed at 25°C for 1 hour.

### -Evaluation of introduction efficiency of fluorescent-labeled amino acid-

9 µL of the 1× sample buffer was added to 1 µL of the reaction solution after the translation reaction. 5 µL thereof was subjected to 15% SDS-PAGE (0.1% SDS). The electrophoresis gel was observed with a fluorescence scanner (Hitachi Software Engineering Co., Ltd., FMBIO-III). The introduction of the fluorescent-labeled amino acid into Polypeptide (1) was confirmed by excitation at 488 nm/detection at 520 nm, and the introduction efficiency was quantified. Table 2 shows relative values in which SEQ ID NO: 1 was set to the reference value of 1.00.

On the other hand, 81 µL of D-PBS (-) (nacalai tesque) and 20 µL of anti-FLAGM2 magnetic beads (Sigma-Aldrich Co., LLC) were added to 9 µL of the reaction solution after the translation reaction, and the mixture was shaken at room temperature for 30 minutes. The magnetic beads were washed 5 times with 200 µL of D-PBS (-). 9 µL of a 2% formic acid aqueous solution was added to the magnetic beads, followed by shaking at room temperature for 1 hour. The supernatant was collected as a purified product of Polypeptide (1). 0.75 µL of the purified solution and 0.75 µL of a saturated α-CHCA solution (50% acetonitrile/0.1% trifluoroacetic acid) were mixed and subjected to mass spectrometry (Bruker, ultrafleXtreme, positive mode measurement). It was confirmed that the fluorescent-labeled amino acid was introduced into Polypeptide (1).

**[Table 2]**

| | SEQ ID NO: 1 | SEQ ID NO: 2 | SEQ ID NO: 3 | SEQ ID NO: 4 |
|---|---|---|---|---|
| | Variant of Mycoplasma capricolum | Variant of Mycoplasma pneumoniae | Variant of Staphylococcus aureus | Variant of Shewanella oneidensis |
| Fluorescent intensity of fluorescent-labeled polypeptide band | 1.00 | 1.11 | 1.00 | 0.70 |

The superiority of the variant of the tRNA for tryptophan of Mycoplasma pneumoniae was shown.

### <Example 2>

A tRNA for tryptophan of Mycoplasma pneumoniae was modified, and tRNAs of SEQ ID NOs: 5 to 9 were designed as tRNAs having UUA in an anticodon. For the tRNAs of SEQ ID NOs: 5 to 9, the production of the tRNA gene, the production of the tRNA, the aminoacylation of the tRNA, the introduction of the fluorescent-labeled amino acid into the polypeptide, and the evaluation of the introduction efficiency were performed by the same methods as in Example 1. Table 3 shows relative values in which SEQ ID NO: 2 was set to the reference value of 1.00.

**[Table 3]**

| | SEQ ID NO: 2 | SEQ ID NO: 5 | SEQ ID NO: 6 | SEQ ID NO: 7 | SEQ ID NO: 8 | SEQ ID NO: 9 |
|---|---|---|---|---|---|---|
| Fluorescent-labeled polypeptide band/entire fluorescence band | 1.00 | 0.79 | 0.83 | 1.04 | 1.15 | 1.05 |

The tRNAs of SEQ ID NO: 7, SEQ ID NO: 8, and SEQ ID NO: 9 had a high introduction efficiency of the fluorescent-labeled amino acid. From this, in the tRNA for tryptophan of Mycoplasma pneumoniae, the superiority of the tRNA in which the combination of a base at a third position from a 5' terminal and a base paired with the base is UA, GU, or UG was shown.

### <Example 3>

A tRNA for tryptophan of Mycoplasma pneumoniae was modified, and tRNAs of SEQ ID NOs: 10 to 14 were designed as tRNAs having UUA in an anticodon. For the tRNAs of SEQ ID NOs: 10 to 14, the production of the tRNA gene, the production of the tRNA, the aminoacylation of the tRNA, the introduction of the fluorescent-labeled amino acid into the polypeptide, and the evaluation of the introduction efficiency were performed by the same methods as in Example 1. Table 4 shows relative values in which SEQ ID NO: 2 was set to the reference value of 1.00.

**[Table 4]**

| | SEQ ID NO: 2 | SEQ ID NO: 10 | SEQ ID NO: 11 | SEQ ID NO: 12 | SEQ ID NO: 13 | SEQ ID NO: 14 |
|---|---|---|---|---|---|---|
| Fluorescent-labeled polypeptide band/entire fluorescence band | 1.00 | 0.71 | 0.57 | 0.86 | 1.03 | 0.83 |

The tRNA of SEQ ID NO: 13 had a high introduction efficiency of the fluorescent-labeled amino acid. From this, in the tRNA for tryptophan of Mycoplasma pneumoniae, the superiority of the tRNA in which the combination of a base at a fourth position from a 5' terminal and a base paired with the base is GU was shown.

### <Example 4>

A tRNA for tryptophan of Mycoplasma pneumoniae was modified, and tRNAs of SEQ ID NOs: 15 to 17 were designed as tRNAs having UUA in an anticodon. For the tRNAs of SEQ ID NOs: 15 to 17, the production of the tRNA gene, the production of the tRNA, the aminoacylation of the tRNA, the introduction of the fluorescent-labeled amino acid into the polypeptide, and the evaluation of the introduction efficiency were performed by the same methods as in Example 1. Table 5 shows relative values in which SEQ ID NO: 2 was set to the reference value of 1.00.

**[Table 5]**

| | SEQ ID NO: 2 | SEQ ID NO: 15 | SEQ ID NO: 16 | SEQ ID NO: 17 |
|---|---|---|---|---|
| Fluorescent-labeled polypeptide band/entire fluorescence band | 1.00 | 1.04 | 0.89 | 1.08 |

The tRNA of SEQ ID NO: 17 had a high introduction efficiency of the fluorescent-labeled amino acid. From this, in the tRNA for tryptophan of Mycoplasma pneumoniae, the superiority of the tRNA in which the combination of a base at a fourth position from a 5' terminal and a base paired with the base is GU and in which the base adjacent to the 5' side of the CCA terminal is G was shown.

### <Example 5>

Formamide was added to the tRNAs of SEQ ID NO: 2 and 5 to 17, subjected to a heat treatment (95°C, 3 minutes), and mixed with a 10× Loading Buffer (Takara, 9157). The mixture was subjected to electrophoresis in a urea-containing acrylamide gel (SuperSep^{™} RNA, 15%, 17well (Wako, 194-15881)) and stained with a staining reagent (Gel Red Nucleic Acid Gel Stain (Biotium, 41003)). Fig. 2 shows an electrophoresis gel after staining. In Fig. 2, the numbers above the gel are sequence numbers.

In the gel of Fig. 2, a change in the higher-order structure of the tRNAs and a sharp band intensity were confirmed in SEQ ID NOs: 7 to 9, 13, and 17, which have high unnatural amino acid introduction efficiency.

### <Example 6>

DNAs (a) to (d) obtained by substituting the base sequence (SEQ ID NO: 21) encoding Polypeptide (1) in DNA (1) were prepared. Each of the DNAs (a) to (d) has a base sequence in which SEQ ID NO: 21 is subjected to the following substitutions.

DNA (a): DNA obtained by substituting the 4th codon with TAG and substituting the eleventh codon with TAA in SEQ ID NO: 21.

DNA (b): DNA obtained by substituting the 4th and 5th codons with TAG and substituting the eleventh codon with TAAin SEQ ID NO: 21.

DNA (c): DNA obtained by substituting the 4th and 6th codons with TAG and substituting the eleventh codon with TAAin SEQ ID NO: 21.

DNA (d): DNA obtained by substituting the 4th and 7th codons with TAG and substituting the eleventh codon with TAAin SEQ ID NO: 21.

The tRNA of SEQ ID NO: 19 was prepared as UAG codon-translating tRNA which is a variant of the tRNA for tryptophan of Mycoplasma capricolum, and N-methylphenylalanine or BODIPYFL-aminophenylalanine was bonded to this tRNA.

The tRNA of SEQ ID NO: 2 was prepared as UAA codon-translating tRNA which is a variant of the tRNA for tryptophan of Mycoplasma pneumoniae, and N-methylphenylalanine or BODIPYFL-aminophenylalanine was bonded to this tRNA.

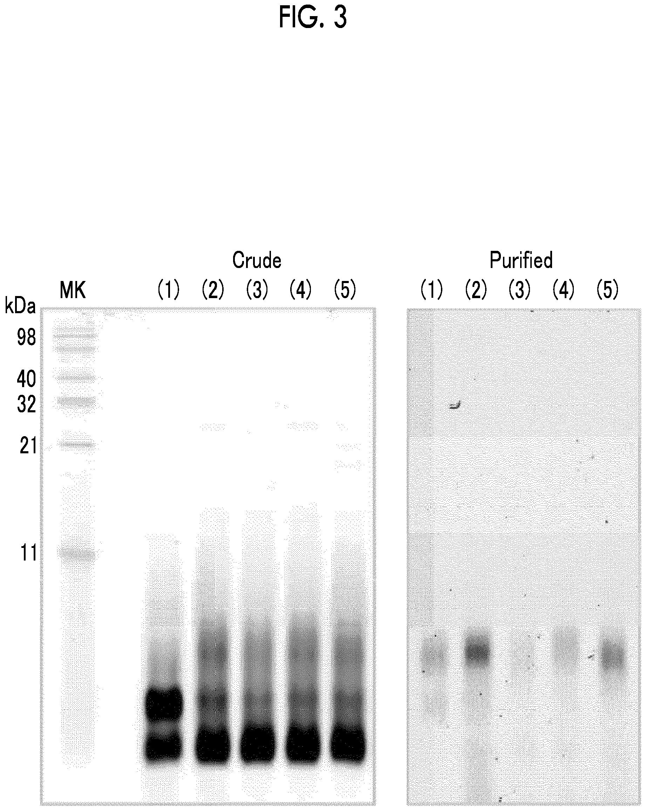

The production of the tRNA gene, the production of the tRNA, the aminoacylation of the tRNA, the introduction of the fluorescent-labeled amino acid into the polypeptide, and the evaluation of the introduction efficiency were performed by the same methods as in Example 1. Fig. 3 shows gels of electrophoresis. The gel on the left side of Fig. 3 is an electrophoresis gel of a specimen before purification, and the gel on the right side of Fig. 3 is an electrophoresis gel of a specimen after purification using a FLAG tag.

In Fig. 3, the numbers on the gel indicate the following polypeptides.
(1) Polypeptide that is a polypeptide translated from DNA (a) using a UAG codon-translating tRNA to which BODIPYFL-aminophenylalanine is bonded and a UAA codon-translating tRNA to which N-methylphenylalanine is bonded.
(2) Polypeptide that is a polypeptide translated from DNA (a) using a UAG codon-translating tRNA to which N-methylphenylalanine is bonded and a UAA codon-translating tRNA to which BODIPYFL-aminophenylalanine is bonded.
(3) Polypeptide that is a polypeptide translated from DNA (b) using a UAG codon-translating tRNA to which N-methylphenylalanine is bonded and a UAA codon-translating tRNA to which BODIPYFL-aminophenylalanine is bonded.
(4) Polypeptide that is a polypeptide translated from DNA (c) using a UAG codon-translating tRNA to which N-methylphenylalanine is bonded and a UAA codon-translating tRNA to which BODIPYFL-aminophenylalanine is bonded.
(5) Polypeptide that is a polypeptide translated from DNA (d) using a UAG codon-translating tRNA to which N-methylphenylalanine is bonded and a UAA codon-translating tRNA to which BODIPYFL-aminophenylalanine is bonded.

In the gel on the right side of Fig. 3, relatively dark band is the polypeptide. As can be seen from the detection of the band of the purified polypeptide, the fluorescent-labeled amino acid and N-methylphenylalanine were able to be multiply introduced into the polypeptide by using two types of tRNA variants.

### <Example 7>

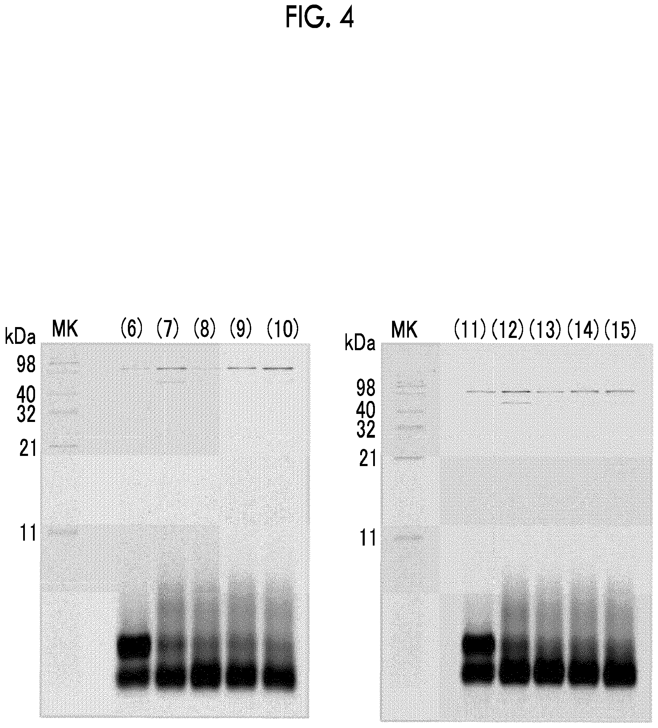

The same experiment as in Example 6 was performed except that puromycin was additionally bonded to the 3' terminal of the mRNAto be translated. N-methylphenylalanine, N-methylalanine, or BODIPYFL-aminophenylalanine was bonded to the UAG codon-translating tRNA and the UAA codon-translating tRNA. Fig. 4 shows a gel of electrophoresis.

In Fig. 4, the numbers on the gel indicate the following polypeptides.
(6) Polypeptide that is a polypeptide translated from DNA (a) using a UAG codon-translating tRNA to which BODIPYFL-aminophenylalanine is bonded and a UAA codon-translating tRNA to which N-methylphenylalanine is bonded.
(7) Polypeptide that is a polypeptide translated from DNA (a) using a UAG codon-translating tRNA to which N-methylphenylalanine is bonded and a UAA codon-translating tRNA to which BODIPYFL-aminophenylalanine is bonded.
(8) Polypeptide that is a polypeptide translated from DNA (b) using a UAG codon-translating tRNA to which N-methylphenylalanine is bonded and a UAA codon-translating tRNA to which BODIPYFL-aminophenylalanine is bonded.
(9) Polypeptide that is a polypeptide translated from DNA (c) using a UAG codon-translating tRNA to which N-methylphenylalanine is bonded and a UAA codon-translating tRNA to which BODIPYFL-aminophenylalanine is bonded.
(10) Polypeptide that is a polypeptide translated from DNA (d) using a UAG codon-translating tRNA to which N-methylphenylalanine is bonded and a UAA codon-translating tRNA to which BODIPYFL-aminophenylalanine is bonded.
(11) Polypeptide that is a polypeptide translated from DNA (a) using a UAG codon-translating tRNA to which BODIPYFL-aminophenylalanine is bonded and a UAA codon-translating tRNA to which N-methylalanine is bonded.
(12) Polypeptide that is a polypeptide translated from DNA (a) using a UAG codon-translating tRNA to which N-methylalanine is bonded and a UAA codon-translating tRNA to which BODIPYFL-aminophenylalanine is bonded.
(13) Polypeptide that is a polypeptide translated from DNA (b) using a UAG codon-translating tRNA to which N-methylalanine is bonded and a UAA codon-translating tRNA to which BODIPYFL-aminophenylalanine is bonded.
(14) Polypeptide that is a polypeptide translated from DNA (c) using a UAG codon-translating tRNA to which N-methylalanine is bonded and a UAA codon-translating tRNA to which BODIPYFL-aminophenylalanine is bonded.
(15) Polypeptide that is a polypeptide translated from DNA (d) using a UAG codon-translating tRNA to which N-methylalanine is bonded and a UAA codon-translating tRNA to which BODIPYFL-aminophenylalanine is bonded.

In the gel of Fig. 4, a thick lower band is BODIPYFL-aminophenylalanine, a slightly upper band is a polypeptide, and a narrow band in the vicinity of 98 kDa is an mRNA-polypeptide conjugate.

As can be seen from the gel of Fig. 4, an mRNA display in which the fluorescent-labeled amino acid and N-methylphenylalanine were multiply introduced was realized.

The disclosure of JP2021-156183 is incorporated in the present specification by reference in its entirety. All literatures, patent applications, and technical standards described in the present specification are incorporated in the present specification by reference to the same extent as in a case where the individual literatures, patent applications, and technical standards are specifically and individually stated to be incorporated by reference.

[Sequence list] International application 21F00967W1JP22035791_5.xml based on International Patent Cooperation Treaty

## Claims

1. A tRNA obtained by modifying a tRNA for tryptophan of Mycoplasma pneumoniae, the tRNA comprising:
a UUA as an anticodon to pair with a UAA codon.

2. The tRNA according to claim 1,
wherein a base adjacent to a 5' side of a CCA terminal is A or G.

3. The tRNA according to claim 1 or 2,
wherein a combination of a base at a third position from a 5' terminal and a base paired with the base is UA, GU, or UG.

4. The tRNA according to claim 1 or 2,
wherein a combination of a base at a fourth position from a 5' terminal and a base paired with the base is GU.

5. A tRNA comprising:
a base sequence set forth in one selected from the group consisting of SEQ ID NO: 7, SEQ ID NO: 8, SEQ ID NO: 9, SEQ ID NO: 13, SEQ ID NO: 15, and SEQ ID NO: 17,
SEQ ID NO: 7: GGUGGUGUAG UUUAGUGGCA GAACAACAGU CUUUAAAACU GUCUGUGUGG GUUCGAUUCC UUCCACCACC ACCA
SEQ ID NO: 8: GGGGGUGUAG UUUAGUGGCA GAACAACAGU CUUUAAAACU GUCUGUGUGG GUUCGAUUCC UUCCACCUCC ACCA
SEQ ID NO: 9: GGUGGUGUAG UUUAGUGGCA GAACAACAGU CUUUAAAACU GUCUGUGUGG GUUCGAUUCC UUCCACCGCC ACCA
SEQ ID NO: 13: GGGGGUGUAG UUUAGUGGCA GAACAACAGU CUUUAAAACU GUCUGUGUGG GUUCGAUUCC UUCCACUCCC ACCA
SEQ ID NO: 15: GGGGGUGUAG UUUAGUGGCA GAACAACAGU CUUUAAAACU GUCUGUGUGG GUUCGAUUCC UUCCACCCCC GCCA
SEQ ID NO: 17: GGGGGUGUAG UUUAGUGGCA GAACAACAGU CUUUAAAACU GUCUGUGUGG GUUCGAUUCC UUCCACUCCC GCCA.

6. An aminoacyl tRNA in which an amino acid is bonded to the tRNA according to any one of claims 1 to 5.

7. The aminoacyl tRNA according to claim 6,
wherein the amino acid is one selected from the group consisting of an unnatural amino acid, a modified amino acid, and derivatives of the unnatural amino acid and the modified amino acid.

8. The aminoacyl tRNA according to claim 6,
wherein the amino acid is one selected from the group consisting of chloroacetyl lysine, N-methylalanine, N-methylphenylalanine, and a fluorescent-labeled amino acid.

9. A reagent for polypeptide synthesis comprising:
a tRNA having CUA as an anticodon to pair with a UAG codon, the tRNA being obtained by modifying a tRNA for tryptophan of Mycoplasma capricolum; and
a tRNA having UUA as an anticodon to pair with a UAA, the tRNA being obtained by modifying a tRNA for tryptophan of Mycoplasma pneumoniae.

10. A reagent for polypeptide synthesis comprising:
an aminoacyl tRNA in which a first unnatural amino acid is bonded to a tRNA having CUA as an anticodon to pair with a UAG codon and being obtained by modifying a tRNA for tryptophan of Mycoplasma capricolum; and
an aminoacyl tRNA in which a second unnatural amino acid is bonded to a tRNA having UUA as an anticodon to pair with a UAA codon and being obtained by modifying a tRNA for tryptophan of Mycoplasma pneumoniae.

11. An introduction method of an unnatural amino acid, which is a method of introducing at least two types of unnatural amino acids into a polypeptide, the introduction method comprising:
expressing a polypeptide from a nucleic acid by using a cell-free peptide synthesis system which contains
a nucleic acid having a base sequence including a UAG codon and a UAA codon,
an aminoacyl tRNA in which a first unnatural amino acid is bonded to a tRNA having CUA as an anticodon to pair with a UAG codon and being obtained by modifying a tRNA for tryptophan of Mycoplasma capricolum; and
an aminoacyl tRNA in which a second unnatural amino acid is bonded to a tRNA having UUA as an anticodon to pair with a UAA codon and being obtained by modifying a tRNA for tryptophan of Mycoplasma pneumoniae.

12. The introduction method of an unnatural amino acid according to claim 11,
wherein the base sequence further includes a UGA codon as a stop codon.

13. A production method of a polypeptide, which is a method of producing a polypeptide in which at least two types of unnatural amino acids are contained in an amino acid sequence, the production method comprising:
expressing a polypeptide from a nucleic acid by using a cell-free peptide synthesis system which contains
a nucleic acid having a base sequence including a UAG codon and a UAA codon,
an aminoacyl tRNA in which a first unnatural amino acid is bonded to a tRNA having CUA as an anticodon to pair with a UAG codon and being obtained by modifying a tRNA for tryptophan of Mycoplasma capricolum; and
an aminoacyl tRNA in which a second unnatural amino acid is bonded to a tRNA having UUA as an anticodon to pair with a UAA codon and being obtained by modifying a tRNA for tryptophan of Mycoplasma pneumoniae.

14. The production method of a polypeptide according to claim 13,
wherein the base sequence further includes a UGA codon as a stop codon.

15. A production method of a nucleic acid display library comprising:
expressing a polypeptide from a nucleic acid by using a cell-free peptide synthesis system to produce a nucleic acid-polypeptide conjugate, the cell-free peptide synthesis system containing
a nucleic acid having a base sequence including a UAG codon and a UAA codon,
an aminoacyl tRNA in which a first unnatural amino acid is bonded to a tRNA having CUA as an anticodon to pair with a UAG codon and being obtained by modifying a tRNA for tryptophan of Mycoplasma capricolum; and
an aminoacyl tRNA in which a second unnatural amino acid is bonded to a tRNA having UUA as an anticodon to pair with a UAA codon and being obtained by modifying a tRNA for tryptophan of Mycoplasma pneumoniae.

16. The production method of a nucleic acid display library according to claim 15,
wherein the production of the nucleic acid-polypeptide conjugate includes producing an mRNA-polypeptide conjugate and reverse-transcribing an mRNA of the mRNA-polypeptide conjugate to produce a cDNA-polypeptide conjugate.

17. The production method of a nucleic acid display library according to claim 15 or 16,
wherein in the nucleic acid-polypeptide conjugate, the nucleic acid and the polypeptide are linked by a puromycin linker.

18. A nucleic acid-polypeptide conjugate comprising:
a polypeptide in which at least two types of unnatural amino acids are contained in an amino acid sequence; and
a nucleic acid having a base sequence encoding the polypeptide, which is linked to the polypeptide,
wherein the base sequence includes a UAG codon and a UAA codon,
in the base sequence, the UAG codon encodes a first unnatural amino acid in the polypeptide, and
in the base sequence, the UAA codon encodes a second unnatural amino acid in the polypeptide.

19. The nucleic acid-polypeptide conjugate according to claim 18,
wherein the nucleic acid-polypeptide conjugate is an mRNA-polypeptide conjugate or a cDNA-polypeptide conjugate.

20. A screening method comprising:
producing a nucleic acid display library by using the production method of a nucleic acid display library according to any one of claims 15 to 17; and
selecting a nucleic acid-polypeptide conjugate having a target activity from the nucleic acid display library and identifying a base sequence of a nucleic acid of the selected nucleic acid-polypeptide conjugate.
